# NEUE EUROPÄISCHE PATENTSCHRIFT

(11) **EP 1 644 306 B2**
(45) Veröffentlichungstag und Bekanntmachung des Hinweises auf die Entscheidung über den Einspruch: **25.12.2019**
(45) Hinweis auf die Patenterteilung: 03.08.2011
(21) Anmeldenummer: 04739287.3
(22) Anmeldetag: 21.05.2004
(51) Int. Cl.: C07C 45/45, C07C 49/255, C07C 45/65

(54) **VERFAHREN ZUR HERSTELLUNG VON ALKENONETHERN**
SIMPLIFIED PRODUCTION OF ALKENONES
PRODUCTION SIMPLIFIEE D'ALCENONES

(30) Priorität: 06.06.2003 DE 10325715
(43) Veröffentlichungstag der Anmeldung: 12.04.2006
(73) Patentinhaber: Solvay Fluor GmbH, 30173 Hannover (DE)
(72) Erfinder: HAUSMANN, Eckhard, 30519 Hannover (DE); BÖSE, Olaf, 30167 Hannover (DE); EICHER, Johannes, 31319 Sehnde-Ilten (DE)
(74) Vertreter: Vande Gucht, Anne
(86) Internationale Anmeldenummer: PCT/EP2004/005466
(87) Internationale Veröffentlichungsnummer: WO 2004/108647

(56) Entgegenhaltungen:
- F. EFFENBERGER ET AL.: "Die Acylierung von Enolethern mit reaktiven Carbonsäurechloriden" CHEMISCHE BERICHTE., Bd. 115, 1982, Seiten 2766-2782, XP002295515 VERLAG CHEMIE GMBH. WEINHEIM., DE
- L, F. TIETZE ET AL.: "Synthesis of Alkyl Propanoates by Haloform Reaction of a Trichloro Ketone" ORGANIC SYNTHESIS., Bd. 69, 1990, Seiten 238-244, XP008037891 USWILEY AND SONS, NEW YORK, NY.
- COLLA A ET AL: "TRIHALOACETYLATED ENOL ETHERS - GENERAL SYNTHETIC PROCEDURE AND HETEROCYCLIC RING CLOSURE REACTIONS WITH HYDROXYLAMINE" SYNTHESIS, GEORG THIEME VERLAG. STUTTGART, DE, 1. Juni 1991 (1991-06-01), Seiten 483-486, XP000196317 ISSN: 0039-7881
- I. I. GERUS ET AL.: "Synthesis and Properties of beta-Ethoxyvinyl Polyfluoroalkyl Ketones" SYNTHESIS., Nr. 5, 2000, Seiten 738-742, XP002295516 GEORG THIEME VERLAG. STUTTGART., DE
- P. P. KLEMCHUK: "Antioxidants: 4. Antioxidant Classes" [Online] 15. Juni 2000 (2000-06-15), WILEY-VCH VERLAG , XP002303289 Gefunden im Internet: URL:http://www.mrw.interscience.wiley.com/ ueic/articles/a03_091/sect4-fs.html DOI: 10.1002/14356007.a03_091> das ganze Dokument

## Beschreibung

Die Erfindung bezieht sich auf ein vereinfachtes Verfahren zur Herstellung von 4-Ethoxy-1,1,1-trifluoro-3-buten-2-on.

Halogenierte Alkenonether, beispielsweise 4-Ethoxy-1,1,1-tritluoro-3-buten-2-on, sind Bausteine in der chemischen Synthese, siehe beispielsweise EP-0 744 400. Man kann sie herstellen, indem man ein Säurechlorid mit einem Vinylether in Anwesenheit einer Base miteinander umsetzt, siehe die obengenannte europäische Offenlegungsschrift, Colla et al. Synthesis, 1991, p. 483-486 und Gerus et al. Synthesis. 2000, p. 738-742; dabei kann die Base auch im Überschuss als Lösemittel eingesetzt werden. Es ist, z. B. aus N. D. Field und D. H. Lorenz, High Polymer, 1970, Seite 394, bekannt, dass die Umsetzung von Phosgen mit Vinylethern in Abwesenheit einer Base zu Polymerisation führt. Aufgabe der vorliegenden Erfindung ist es, ein vereinfachtes Verfahren zur Herstellung von Alkenonen anzugeben. Diese Aufgabe wird durch das Verfahren der vorliegenden Erfindung gelöst.

Das weiter unten definierte erfindungsgemäße Verfahren zur Herstellung von Alkenonen sieht vor, dass man Vinylether und Carbonsäurechlorid, in Abwesenheit eines Säurefängers miteinander umsetzt. Der Begriff "Säurefänger" umfasst insbesondere Basen, besonders Stickstoffbasen wie Pyridin oder sekundäre und tertiäre Amine sowie Onium-Salze, die in der nicht vorveröffentlichten internationalen Patentanmeldung mit der Anmeldenummer PCT/EP 03/00913 beschrieben sind, wobei mit dieser Bezeichnung kein Erklärungsversuch verbunden sein soll.

Die Erfindung betrifft ein Verfahren zur Herstellung von Alkenonen umfassend die Anlagerung von Carbonsäurehalogeniden an Vinylether und anschließende Halogenwasserstoffabspaltung, wobei man Alkenon der Formel (I) herstellt

R¹-C(O)-C(H)=C(H)-OR² (I)

wobei R¹ für CF₃ steht und R² für Ethyl steht, wobei man ein Säurehalogenid der Formel (II)

R¹-C(O)X, (II)

worin X für Cl steht und R¹ die obengenannte Bedeutung besitzt, mit einem Vinylether der Formel (III)

CH₂ = C(H)-OR² (III)

worin R² die obengenannte Bedeutung besitzt miteinander umsetzt, dadurch gekennzeichnet, dass man die Umsetzung in Abwesenheit eines Säurefängers für entstehenden Halogenwasserstoff durchführt, und wobei man die Umsetzung zweistufig durchführt, wobei die erste Stufe die Anlagerung des Säurehalogenids an den Vinylether und die 2. Stufe die Halogenwasserstoffabspaltung betrifft, und wobei die Temperatur in der 2. Stufe im Bereich von bis zu 150 °C liegt.

Das Molverhältnis von Säurechlorid und Vinylether liegt zweckmäßig zwischen 0,8:1 und 1:0,8, besonders zwischen 0,8:1 bis 1:1.

Das erfindungsgemäße Verfahren wird zweistufig durchgeführt. In der 1. Stufe wird das Säurechlorid an den Vinylether addiert. Die Reaktion kann exotherm sein, so dass das Reaktionsgemisch gegebenenfalls gekühlt werden muß oder die Umsetzung sehr langsam durchgeführt wird. Es ist vorteilhaft, einen Kühleraufsatz zu verwenden, der das Säurechlorid kondensiert und eine Rückführung in das Reaktionsgemisch ermöglicht. Diese Stufe wird zweckmäßig bei -15 °C bis +50 °C, vorzugsweise -15 °C bis +30 °C durchgeführt. Die 2. Stufe umfasst die Elemination von Chlorwasserstoff. Sie wird zweckmäßig bei der zur Elemination notwendigen Temperatur durchgeführt und kann leicht ermittelt werden, indem man die Abspaltung des Chlorwasserstoffs beobachtet. Bei der Umsetzung von Trifluoracetylchlorid an Ethylvinylether liegt sie im Bereich von bis zu 150 °C, vorzugsweise 30 °C bis 90 °C.

Bevorzugt verwendet man bei der erfindungsgemäßen Reaktion zwischen Säurechlorid und Vinylether kein Lösungsmittel. Vorteil ist, dass kein Lösungsmittel abgetrennt werden muß (kein Aufwand für Rückgewinnung nötig, geringerer Energiebedarf).

Das erfindungsgemäße Verfahren zur Herstellung von Alkenonen der Formel (I) kann bei erhöhtem Druck durchgeführt werden. Umgebungsdruck oder leichter Unterdruck (bis herab zu 0,5 bar) ist von Vorteil, weil entstandener Halogenwasserstoff besser aus der Reaktionsmischung entfernt werden kann. Es kann batchweise oder teilkontinuierlich durchgeführt werden. Entstehender Halogenwasserstoff kann während oder nach der Umsetzung z. B. durch Erwärmen oder Unterdruck oder beides aus der Reaktionsmischung entfernt werden.

Das erfindungsgemäße Verfahren umfaßt die Umsetzung in Abwesenheit eines Säurefängers (dieser Begriff umfaßt auch Oniumsalze), und in Abwesenheit des Säurefängers und Anwesenheit eines Stabilisators. Als Stabilisatoren sind z. B mit mehreren Alkylgruppen substituierte Phenole, z. B. durch 2 t-Butylgruppen und eine Alkylgruppe mit 1 bis 3 C-Atomen substituiertes Phenol, insbesondere 2,6-Di-t-butyl-4-methylphenol geeignet.

Die Umsetzung in Abwesenheit eines Säurefängers, bzw. Umsetzung in Abwesenheit eines Säurefängers und Anwesenheit eines Stabilisators für das herzustellende Alkenon, sind sehr viel vorteilhafter als das Verfahren des Standes der Technik, weil der Säurefänger nicht abgetrennt werden muß. Diese beiden Varianten werden weiter erläutert.

Die eine Variante sieht die Umsetzung der Ausgangsverbindungen generell in Abwesenheit eines Säurefängers für das Alkenon vor. Der Begriff "Säurefänger" ist weiter oben definiert.

Die andere der beiden Varianten sieht vor, die Ausgangsverbindungen in Abwesenheit eines Säurefängers, aber in Anwesenheit eines Stabilisators für das herzustellende Alkenon umzusetzen. Als Stabilisatoren sind z. B die oben schon genannten, mit mehreren Alkylgruppen substituierte Phenole, z. B. durch 2 t-Butylgruppen und eine Alkylgruppe mit 1 bis 3 C-Atomen substituiertes Phenol, insbesondere 2,6-Di-t-butyl-4-methylphenol.

Die Aufarbeitung der Reaktionsgemische erfolgt nach üblichen Methoden. Beispielsweise kann man das gewünschte Alkenon der Formel (I) aus dem Gemisch herausdestillieren.

Vorteil des erfindungsgemäßen Verfahrens ist, dass durch die Abwesenheit eines Säurefängers die Aufarbeitung erleichtert ist. Die Anwesenheit eines Stabilisators für das Alkenon bereits in der Reaktionsmischung hilft die Ausbeute zu erhöhen. Die Abwesenheit eines Säurefängers bei gleichzeitiger Anwesenheit eines Stabilisators für das Alkenon in der Reaktionsmischung kann sehr günstig für die Ausbeute sein.

Die folgenden Beispiele sollen die Erfindung weiter erläutern, ohne sie in ihrem Umfang einzuschränken.

### Beispiele

### Beispiel 1:

Umsetzung von Ethylvinylether und Trifluoracetylchlorid zu 4-Ethoxy-1,1,1-trifluoro-3-buten-2-on ("ETFBO") in Abwesenheit eines Säurefängers und Anwesenheit eines Stabilisators für das herzustellende Alkenon.

### Ansatz:

0,25 g (1,13 mmol) 2,6-Di-t-butyl-4-methylphenol ("BHT")
12,9 g (99 %, 0,18 mol) Ethylvinylether ("EVE")
21,0 g (0,16 mol) Trifluoracetylchlorid ("TFAC")

### Durchführung:

BHT und EVE wurden miteinander vermischt. Im Eisbad wurde dann unter Trockeneisrückflusskühlung das TFAC eingeleitet und dabei die Temperatur der Reaktionsmischung bei unterhalb von 26 °C gehalten. Anschließend wurde das Reaktionsgemisch bei 80 °C thermolysiert. Nach der Thermolyse wog das Reaktionsgemisch noch 28,5 g. Es wurde dann bei 9 mbar destilliert.

### Ausbeute:

24,1 g = 83,0 % d.Th.

Eine Wiederholung mit 0,25 g BHT, 13,8 g EVE und 19,7 g TFAC ergab eine Ausbeute von 87,6 % d. Th.

### Beispiel 2:

Herstellung von ETFBO ohne Stabilisator

### Ansatz:

13,0 g (99 %, 0,19 mol) EVE
19,1 g (0,14 mol) TFAC

Das EVE wurde vorgelegt und bei Raumtemperatur unter Trockeneiskühlung das TFAC zugegeben. Anschließen wurde bei 80 °C thermolysiert. Das thermolysierte Gemisch wurde dann bei 7 mbar destilliert.

### Ausbeute:

22,8 g = 90,4 % d. Th.

## Patentansprüche

1. Verfahren zur Herstellung von Alkenonen umfassend die Anlagerung von Carbonsäurehalogeniden an Vinylether und anschließende Halogenwasserstoffabspaltung, wobei man Alkenon der Formel (I) herstellt
R¹-C(O)-C(H)=C(H)-OR² (I)
wobei R¹ für CF₃ steht und R² für Ethyl, wobei man ein Säurehalogenid der Formel (II)
R¹-C(O)X, (II)
worin X für Cl, steht und R¹ die obengenannte Bedeutung besitzt, mit einem Vinylether der Formel (III)
CH₂ = C(H)-OR² (III)
worin R² die obengenannte Bedeutung besitzt miteinander umsetzt, **dadurch gekennzeichnet, dass** man die Umsetzung in Abwesenheit eines Säurefängers für entstehenden Halogenwasserstoff durchführt, und wobei man die Umsetzung zweistufig durchführt, wobei die erste Stufe die Anlagerung des Säurehalogenids an den Vinylether und die 2. Stufe die Halogenwasserstoffabspaltung betrifft, und wobei die Temperatur in der 2. Stufe im Bereich von bis zu 150 °C liegt.

2. Chemisches Syntheseverfahren umfassend (a) die Herstellung eines Alkenons der Formel
R¹-C(O)-C(H)=C(H)-OR² (I)
wobei R¹ für CF₃ steht, gemäss dem Verfahren nach Anspruche 1 und (b) die Verwendung des Alkenons als Baustein in der chemischen Synthese.

## Claims

1. Process for producing alkenones encompassing the formation of an adduct of acyl halides with vinyl ethers and subsequent elimination of hydrogen halide, where alkenone of the formula (I) is produced
R¹-C(O)-C(H)=C(H)-OR² (I)
where R¹ is CF₃ and R² is ethyl, where an acyl halide of the formula (II)
R¹-C(O)X, (II)
in which X is Cl and the definition of R¹ is as above, is reacted with a vinyl ether of the formula (III)
CH₂ = C(H)-OR² (III)
in which the definition of R² is as above, **characterized in that** the reaction is carried out in the absence of any acid scavenger for hydrogen halide produced, wherein the process is carried out in two steps, wherein in the first step, the acid halide is added to the vinyl ether, wherein the second step comprises the elimination of hydrogen halide, and wherein the temperature in the second step lies in the range of up to 150°C.

2. Chemical synthesis process encompassing (a) the production of an alkenone of the formula
R¹-C(O)-C(H)=C(H)-OR² (I)
where R¹ is CF₃ in accordance with the process according to claim 1 and (b) the use of the alkenone as unit in chemical synthesis.

## Revendications

1. Procédé pour la préparation d'alcénones, comprenant la formation d'un adduit d'halogénures d'acyles avec des éthers vinyliques et l'élimination subséquente d'hydracide halogéné, dans lequel on prépare une alcénone de formule (I)
R¹-C(O)-C(H)=C(H)-OR² (I)
R¹ représentant un groupe CF₃ et R² représentant un groupe éthyle, dans lequel on fait réagir l'un avec l'autre un halogénure d'acyle de formule (II)
R¹-C(O)X, (II)
dans laquelle X représente Cl et R¹ a la signification donnée ci-dessus, avec un éther vinylique de formule (III)
CH₂=C(H)-OR² (III)
dans lequel R² a la signification donnée ci-dessus, **caractérisé en ce qu'**on effectue la réaction en absence d'un capteur d'acide pour l'hydracide halogéné résultant dans lequel le procédé est mis en œuvre en deux étapes, dans lesquelles, dans la première étape, l'halogénure d'acyle est ajouté à l'éther vinylique, dans lequel la deuxième étape comprend l'élimination de l'hydracide halogéné et dans lequel la température dans les deuxième étape est comprise dans l'intervalle allant jusqu'à 150°C.

2. Procédé de synthèse chimique, comprenant (a) la préparation d'une alcénone de formule
R¹-C(O)-C(H)=C-(H)-OR² (I)
dans laquelle R¹ représente un groupe CF₃, conformément au procédé selon le revendication 1 (b) l'utilisation de l'alcénone comme composant en synthèse chimique.
